**Europäisches Patentamt**

**European Patent Office**

**Office européen des brevets**

(19)

(11) Veröffentlichungsnummer: **0 265 848 B1**

(12) # EUROPÄISCHE PATENTSCHRIFT

(45) Veröffentlichungstag der Patentschrift: **25.09.91**

(51) Int. Cl.⁵: **A61K 47/00, A61K 31/44**

(21) Anmeldenummer: **87115516.4**

(22) Anmeldetag: **22.10.87**

(54) Arzneizubereitungen zur oralen Verabreichung, die als Einzeldosis 10 bis 240 mg Dihydropyridin enthalten.

(30) Priorität: **23.10.86 DE 3636123**

(43) Veröffentlichungstag der Anmeldung:
**04.05.88 Patentblatt 88/18**

(45) Bekanntmachung des Hinweises auf die
Patenterteilung:
**25.09.91 Patentblatt 91/39**

(84) Benannte Vertragsstaaten:
**AT BE CH DE ES FR GB IT LI LU NL SE**

(56) Entgegenhaltungen:
EP-A- 0 068 450
LU-A- 65 929

H.P. FIEDLER, Lexicon der Hilfsstoffe, Editor
Cantor Verlag 1981, S. 386-387

(73) Patentinhaber: **Dr. Rentschler Arzneimittel
GmbH & Co.
Postfach 320 Mittelstrasse 18
W-7958 Laupheim(DE)**

(72) Erfinder: **Lahr, Wolfgang
Silcherweg 29
W-7958 Laupheim(DE)**
Erfinder: **Köhne, Hans, Dr. Dipl.-Chem.
Rotbachweg 7
W-7958 Obersulmetingen(DE)**
Erfinder: **Musculus, Frank
Gerbe 2
W-7958 Achstetten(DE)**
Erfinder: **Schmersahl, Hein-Uwe, Dr.
Händelstrasse 36
W-6479 Limesharn 1(DE)**

(74) Vertreter: **Schwabe - Sandmair - Marx
Stuntzstrasse 16
W-8000 München 80(DE)**

## Beschreibung

Die Erfindung betrifft neue feste Arzneizubereitungen zur oralen Verabreichung und ihre Verwendung zur Behandlung von Erkrankungen des Blutkreislaufes, die Dihydropyridine in gelöstem, molekulardispersem Zustand in PEG mit Molgewichten von 6 000 bis 35 000 zusammen und mit einem Fettalkohol oder Fettalkoholgemisch mit Kettenlängen von 6 bis 30 Kohlenstoffatomen enthalten.

Die Herstellung von Dihydropyridinen wie z. B. Nifedipin, als auch nifedipinverwandter Substanzen ist aus der GB-PS 11 73 862 bekannt Dihydropyridine werden zur Behandlung von Blutkreislauferkrankungen und des Bluthochdruckes verwendet und sind hochwirksame Substanzen, die wegen ihrer Nebenwirkungen je nach Krankheitsbild und individuellem Substanzbedarf dem Patienten angepaßt verabreicht werden müssen. Aus der GB-PS 11 73 862 geht beispielsweise hervor, daß Nifedipin, d. i. 4-(2-Nitrophenyl)-2,6-dimethyl-3,5-dicarbomethoxy-1,4-dihydropyridin, in einer Dosis von 2,5 mg pro Patient oral zu verabreichen sei. Bei der Behandlung der Angina pectoris sollen, bezogen auf 70 kg schwere Patienten, drei Dosen pro Tag gegeben werden. In Abhängigkeit von der Reaktion der Patienten kann diese Dosis erhöht oder verringert werden. Wenn größere Dosen verabreicht werden, sollen sie über mehrere Einzeldosen gegeben werden. Bekannte Nebenwirkungen sind Übelkeit, Schwindel, Müdigkeit, Hautreaktionen, Kribbeln in Armen und Beinen, Blutdrucksenkung unter die Norm, Herzklopfen und Erhöhung der Pulsfrequenz. Nifedipin und seine Derivate besitzen biologische Halbwertszeiten, die unter pathophysiologischen Gegbenheiten unterschiedlich sein können. So werden für Nifedipin Eliminationshalbwertszeiten von 2 bis 3 Stunden bis zu 4 bis 11 Stunden angegeben. Für andere Dihydropyridine wie Nitrendipin werden 6 bis 15 Stunden, für Nimodipin 1,5 bis 2 Stunden und bis zu 22 Stunden bei chronischer Niereninsuffizienz bei einer Kreatinin Clearance < 30 ml/Minute genannt.

Diese biopharmazeutischen Daten lassen den Fachmann erkennen, daß es beispielsweise für Nifedipin und seine Derivate problematisch ist, einen Gleichgewichtszustand im Organismus (steady state) zwischen absorbierter und eleminierter Wirkstoffmenge herzustellen. Insbesondere bedarf es für jeden Patienten einer individuellen Dosiseinstellung, um therapeutisch wirksame Blutspiegel zu erzeugen. Dabei ist es erforderlich so zu dosieren, daß ein vertretbares Verhältnis von Wirkung zu Nebenwirkung eingehalten wird.

Dies wird beispielsweise unvollkommen für Nifedipin durch bekannte initial freisetzende Arzneiformen (Beißkapseln DE-PS 22 09 529) erreicht. Diese initial wirkenden Darreichungsformen lassen den Wirkstoff im Blut rasch auf ein hohes Niveau zu Spitzenkonzentrationen anfluten, die in der Regel nicht zur Therapie benötigt werden, um wiederum rasch in subtherapeutische Konzentration abzufallen (Dtsche Apoth. Ztg. 125 (1985), S. 1174 - 1176, Abb. 1). Der unteren therapeutisch wirksamen Plasmakonzentration von 10 - 15 Nanogramm pro Milliliter (Selecta 10 (1983), S. 860) stehen in der vorgenannten Veröffentlichung bis zu 185 Nanogramm pro Milliliter gegenüber. Daraus resultiert, daß spätestens nach 3 bis 4 Stunden erneut eine Dosis verabreicht werden muß. Offensichtlich ist durch initial freisetzende Formen dieser Art eine Therapiesicherheit nur durch viele Einzelgaben gewährleistet.

Arzneistoffe, die zur therapiegerechten Behandlung täglich mehrmals verabreicht werden müssen, werden häufig in Zubereitungen mit retardierter, d. h. verzögerter Freisetzungscharakteristik überführt. Eine Retardierung ist dann sinnvoll, wenn die Eliminationshalbwertszeit des Wirkstoffes ausreichend kurz ist und/oder durch diese Maßnahme die Regelmäßigkeit der Einnahme (Patienten compliance) verbessert werden soll. Ziel einer Retardierung ist es, daß sich nach mehrmaliger Gabe ein gleichmäßiger therapeutisch wirksamer Blutspiegel mit möglichst geringen Schwankungen zwischen $C_{max}$ und $C_{min}$ (d. i. maximale und minimale Blutspiegelkonzentration) ausbildet.

So sind auch für Nifedipin und seine Derivate verschiedene Retardformen und Verfahren zu ihrer Herstellung bekannt.

Die GB-PS 20 53 681 beschreibt für die Dihydropyridine Nicardipin und Nifedipin Retardformulierungen, in denen die jeweilige Substanz in amorpher Form zusammen mit Polyethylenoxid und weiteren Hilfsstoffen vorliegt.

In der DE-OS 30 24 858, dem deutschen Gegenstück zur GB-PS 20 53 681 werden ergänzende Ergebnisse einer Bioverfügbarkeitsstudie am Hund vorgelegt, die zwar gegenüber dem Vergleich eine verbesserte bioverfügbarkeit zeigen, jedoch auch deutlich erkennen lassen, daß die Blutspiegel nach spätestens 6 Stunden abfallen.

Die GB-PS 21 59 407 beschreibt eine feste Nifedipinzubereitung unter Verwendung von Casein und anorganischen Hilfsstoffen, wobei aus der Bioverfügbarkeitsprüfung hervorgeht, daß gleichmäßige Blutspiegel über einen Zeitraum von 4 bis 6 Stunden erreicht werden, gefolgt von einem raschen Abfall (Fig. 7).

Die EP-A-00 47 899 lehrt die Herstellung nifedipinhaltiger fester Arzneizubereitungen, in denen der Wirkstoff mit einer definierten spezifischen Oberfläche vorliegt, wobei man sich die Selbstretardierung praktisch wasserunlöslicher Nifedipinkristalle zunutze macht (langsames Lösen der Kristalle).

EP 0 265 848 B1

Die angegebenen Plasmakonzentrationen zeigen für Beispiel 1 der EP-A-00 47 899 eine rasche Anflutung nach der ersten Stunde und einen plateauartigen Verlauf für die zweite bis achte Stunde, gefolgt von einem raschen Absinken auf ein niedrigeres Niveau. Der Verlauf für Beispiel 2 zeigt von der ersten bis zur sechsten Stunde ein Plateau mit anschließendem Abfall.

Aus der LU-A-65 929 ist eine Gelatinebeißkapsel bekannt, die in beliebiger Reihenfolge Nifedipin, einen Polyalkylenglykol mit 2 bis 3 C-Atomen in den Alkylenresten und einem durchschnittlichen Molekulargewicht von 200 bis 4000, einen niederen Alkohol mit 2 bis 8 C-Atomen und gegebenenfalls weitere Formulierungshilfsmittel enthält.

Aus der EP-A 0 068 450 sind Arzneimittel zur oralen Verabreichung bekannt, bei denen der Wirkstoff in eine wasserunlösliche, schmelzbare Komponente aus Fettalkohol mit Kettenlängen von 12 bis 24 Kohlenstoffatomen eingebettet ist.

Darüberhinaus wird der Stand der Technik aus den auf dem Markt befindlichen Präparaten und ihren Dosierungsempfehlungen gebildet. Alle bekannten Nifedipin-Retard Präparate sind nach den Herstellerangaben mit zweimal täglich einer bis viermal täglich einer Tablette angegeben mit einem Einnahmeabstand von 4 bis 12 Stunden. Das Nifedipinderivat Nimodipin wird als Nimotop® oral in sechsstündigem Abstand 4mal täglich mit 2 Tabletten zu 30 mg verabreicht (Rote Liste 1986, lfd. Nr. 26084).

Für handelsübliche nifedipinhaltige Retardformen wird anhand einer Bioverfügbarkeitsstudie angezeigt, daß trotz Retardierung der Wirkstoff zu Spitzenkonzentrationen rasch ansteigt (die weit über den therapeutisch notwendigen Konzentrationen liegen) und spätestens nach 10 Stunden den minimalen therapeutischen Wirkspiegel unterschreiten (Arzneim.-Forsch./Drug Res. 35 (II), Nr 12, 1983, S. 1840 - 1842).

Aus diesen Angaben und Meßergebnissen geht hervor, daß es zwar möglich ist Nifedipin und seine verwandten Substanzen zu retardieren, um über längere Zeit anhaltende Blutspiegelkonzentrationen zu erhalten, doch sind diese Versuche unvollkommen geblieben, wie die praktische Anwendung zeigt.

Es war deshalb überraschend festzustellen, daß es möglich ist, Dihydropyridine für die orale Verabreichung so zu retardieren, daß nach Anwendung derartiger Präparate am Menschen gleichmäßige, d. h. plateauartige Blutspiegelverläufe über mehr als 12 Stunden, daß heißt als 24 Stunden gemessen werden können.

Es war darüberhinaus überraschend, daß derartige Arzneizubereitungen mit Wirkstoffmengen, die weit über den bisher üblichen Dosen liegen, nämlich in Mengen von 10 bis 240 mg, vorzugsweise 30 bis 120 mg, insbesondere 40 bis 90 mg pro Einzeldosierung, verabreit werden können, ohne daß es nach Einnahme zu den vorbeschriebenen Nebenwirkungen kommt, die diesen Substanzen, insbesondere bei Blutspiegelspitzenkonzentrationen, eigen sind.

Überraschend war ferner, daß der Wirkstoff aus den erfindungsgemäßen Arzneizubereitungen je nach quantitativer und qualitativer Zusammensetzung der Hilfsstoffe, im Organismus so langsam anflutet, daß ausgeprägte Blutspiegelmaxima, d.h. Blutspiegelspitzen vermieden werden und dennoch therapeutisch wirksame Blutspiegel erreicht werden.

Es war weiterhin nicht zu erwarten, daß die vorbeschriebenen Effekte der erfindungsgemäßen Arzneizubereitungen eintreten, da der Wirkstoff in vitro ein für konventionelle Retardformen übliches Freisetzungsverhalten zeigt.

Neu war auch die Erkenntnis und für den Fachmann überraschend, daß diese vorteilhaften Effekte auftreten, wenn die erfindungsgemäß verwendbaren Dihydropyridine in gelöstem, molekulardispersem Zustand, d. h. als feste Lösung in der Darreichungsform vorliegen. Es wäre zu erwarten gewesen, daß die an sich praktisch unlöslichen Substanzen im nichtkristallinen Zustand besonders schnell resorbiert werden, wie dies aus der EP-A-01 67 909 hervorgeht und anschließend entsprechend schnell eliminiert werden. Ein typisches Beispiel für eine schnell ansteigende Blutspiegelkonzentration wird in der DE-OS 33 26 167 beschrieben. Das dort verwendete Glibenclamid ist ähnlich den erfindungsgemäß verwendeten Dihydropyridinen praktisch wasserunlöslich. Durch Überführung dieser Substanz in den nichtkristallinen Zustand wird dort eine rasche Anflutung des Wirkstoffes im Blutplasma erreicht, mit den dort gewünschten Spitzenkonzentrationen.

Die erfindungsgemäßen Arzneizubereitungen enthalten im wesentlichen das zu retardierende Dihydropyridin, Polyethylenglykol (e) und einen Fettalkohol bzw. ein Fettalkoholgemisch als formgebende Matrix. Als Lösungsmittel des Dihydropyridins werden vorzugsweise Polyethylenglykole mit Molekulargewichten von 6000 bis ca. 35 000 verwendet. Zur Verbesserung der Löslichkeit des Dihydropyridins in dem Lösungsmittel Polyethylenglykol können ein oder mehrere lösungsvermittelnde Hilfsstoffe zugegeben werden, insbesondere dann, wenn hochmolekulare Polyethylenglykole verwendet werden oder das Dihydropridin zur Rekristallisation in der Arzneiform neigt.

Die Dihydropyridine liegen pro Darreichungsform erfindungsgemäß in Mengen von 10 bis 240 mg, vorzugsweise 30 bis 120 mg, insbesondere 40 bis 80 mg vor, wobei als Dihydropyridine neben Nifedipin,

3

nifedipinähnliche Substanzen wie z. B. Nimodipin, Nitrendipin, Nicardipin, Nisoldipin und Felodipin verwendet werden.

Die verwendeten Polyethylenglykole mit Molekulargewichten von 6000 bis ca. 35 000 werden entweder als einheitlich anzusehende Molekularfraktionen oder in Form von Mischungen verschiedener Molekularfrationen eingesetzt, wobei das bevorzugte Massenverhältnis Dihydropyridin zu Polyethylenglykol 1:2 bis 1:50, insbesondere 1:4 bis 1:40 beträgt. Als matrixgebende Fettalkohole werden vornehmlich gesättigte Fettalkohole verwendet, die bei Raumtemperatur fest oder flüssig sein können, d. h. solche mit Kettenlängen von 6 bis 30 Kohlenstoffatomen. Dabei werden Massenverhältnisse des Dihydropyridins zu Fettalkohol von 1:0,1 bis 1:10 insbesondere 1:1 bis 1:3 bevorzugt.

Um feste Arzneiformen zu erhalten, werden bei der Verwendung von flüssigen Fettalkoholen bei Raumtemperatur feste Polyethylenglykole verwendet und umgekehrt. Es ist jedoch möglich, feste Fettalkohole mit festen Polyethylenglykolen zu mischen. Die Mischungen werden erforderlichenfalls oberhalb der Schmelztemperatur hergestellt.

Als für die Löslichkeitsverbesserung des Dihydropyridins in den Polyethylenglykolen infrage kommenden lösungsvermittelnden Hilfsstoffe sind alle die geeignet, die sich nicht stabiliätsmindernd auf die erfindungsgemäßen Formen auswirken und als pharmakologisch verträglich anzusehen sind. Derartige Substanzen sind bekanntermaßen Polyvinylpyrrolidone, Polyoxyethylenfettalkohol ether, Polyoxyethylensorbitanfettsäureester Polyoxvethylenstearinsäureester, Pluronics®, Fettakoholsulfate und andere Tenside. Werden derartige lösungsvermittelnde Substanzen verwendet, dann beträgt das Massenverhältnis von Dihydropyridin zu Lösungsvermittler von 1 : 0,01 bis 1 : 3.

Darüber hinaus können die erfindungsgemäßen Arzneizubereitungen weitere Hilfsstoffe wie z. B. Farbstoffe, Gleit- und Schmiermittel, Sprengmittel, Füllstoffe, Weichmacher und ähnliches enthalten.

Die Herstellung der erfindungsgemäßen Zubereitungen erfolgt nach an sich bekannten Verfahren, indem das Dihydropyridin in dem ausgewählten Polyethylenglykol, gegebenenfalls in der Wärme, gelöst wird. Zu dieser Mischung wird der Fettalkohol zugegeben, so daß in der Regel eine klare Schmelze vorliegt. Gegebenenfalls fügt man eine lösungsvermittelnde Komponente und wahlweise andere Hilfsstoffe zu. Diese Mischungen sind bei Raumtemperatur hochviskos bis fest und können so zu Darreichungsformen verarbeitet werden. Bevorzugte Darreichungsformen der erfindungsgemäßen Arzneizubereitungen sind Tabletten, Kapseln, Pillen, überzogene Tabletten, Sachets, mehrschichtige Tabletten und Granulate, deren Herstellung nach an sich bekannten Verfahren erfolgt. Da Dihydropyridine lichtempfindlich sind, werden alle Arbeiten unter Lichtschutz durchgeführt.

Gegebenenfalls sind die erfindungsgemäßen Arzneizubereitungen mit anderen therapeutisch sinnvollen Wirksubstanzen, wie z. B. α-Blockern, Betablockern, Diuretika zu kombinieren beispielsweise in Mehrschichttabletten, Kapseln, u.s.w.. Sofern es therapeutisch gewünscht ist, lassen sich auch mehrere der beispielhaft genannten Dihydropyridine miteinander kombinieren.

Nachfolgende Beispiele sollen die Zusammensetzung und Verfahren zur Herstellung der erfindungsgemäßen Zubereitungen anhand von Nifedipin als exemplarischen Dihydropyridin erläutern:

Beispiel 1

| | |
|---|---|
| **Nifedipin** | **10,0 g** |
| **Polyethylenglykol** | |
| **(Mittl.Mol.-Gewicht 200)** | **80,0 g** |
| **Polyethylenglykol** | |
| **(Mittl.Mol.-Gew. 6000)** | **30,0 g** |
| **Stearylalkohol** | **30,0 g** |
| **Polyvinylpyrrolidon** | **5,0 g** |

Die beiden Polyethylenglykole werden unter Erwärmen miteinander gemischt und das Nifedipin unter Rühren darin gelöst. In die Schmelze wird der Stearylalkohol gegeben und gleichfalls geschmolzen. Nach Zugabe des Polyvinylpyrrolidons wird die noch flüssige Masse in Hartgelatinekapseln Größe 3 zu 310 mg, entsprechend 20 mg Nifedipin, abgefüllt.

Beispiel 2

| Nifedipin | 10 g |
|---|---|
| Polyethylenglykol (Mittl.Mol.-Gewicht 200) | 90 g |
| Polyethylenglykol (Mittl.Mol.-Gewicht 20,000) | 25 g |
| Stearylalkohol | 30 g |

Die Herstellung erfolgt analog Beispiel 1. Die Schmelze wird in Hartgelatinekapseln Größe 1 zu 465 mg, entsprechend 30 mg Nifedipin, abgefüllt.

Beispiel 3

| Nifedipin | 10 g |
|---|---|
| Polyethylenglykol (Mittl.Mol.-Gewicht 20,000) | 90 g |
| Octylalkohol | 15 g |

Das Nifedipin wird in dem geschmolzenen Polyethylenglykol eingerührt und gelöst. Anschließend wird der Fettalkohol zugegeben. Die Schmelze wird in Hartgelatinekapseln Größe 0 zu 620 mg, entsprechend 40 mg Nifedipin, abgefüllt.

Beispiel 4

| Nifedipin | 40 g |
|---|---|
| Polyethylenglykol (Mittl.Mol.-Gewicht 6000) | 440 g |
| Stearylalkohol | 120 g |
| Polyvinylpyrrolidon | 20 g |

Die Herstellung der Schmelze erfolgt analog Beispiel 1. Anschließend wird die Schmelze ausgegossen und nach dem Erstarren mittels einer Siebmaschine auf einen oberen Partikeldurchmesser von 1,0 mm zerkleinert.

Das gemahlene Pulver wird mit je 1 Gew.-% Magnesiumstearat und kolloidaler Kieselsäure vermischt. Die Mischung wird zu Oblong-Tabletten mit einem Gehalt von 60 mg Nifedipin verpresst.

In vitro Freisetzungsraten

Das Freisetzungsverhalten des Wirkstoffes aus den Arzneiformen wurde in Anlehnung an die US-Pharmakopoe geprüft. Der pH-Wert des Freisetzungsmediums wurde konstant bei 1,5 gehalten. (Angaben in % der jeweils enthaltenen Wirkstoffmenge)

Als Vergleich wurde eine handelsübliche Retardtablette (Adalat® retard, 20 mg) verwendet.

Tabelle 1

|  | Beisp. 1 | Beisp. 2 | Beisp. 3 | Beisp. 4 | Vergleich |
|---|---|---|---|---|---|
| 1 h | 13 % | 15 % | 19 % | 21,5 % | 23 % |
| 2 h | 22 % | 23 % | 25 % | 41 % | 45 % |
| 3 h | 34,5 % | 28 % | 36 % | 55 % | 57 % |
| 4 h | 48 % | 34 % | 47 % | 65 % | 66 % |
| 5 h | 60 % | 44 % | 56 % | 72 % | 71 % |
| 6 h | 69 % | 53 % | 68 % | 77 % | 74 % |
| 7 h | 81 % | 62 % | 79 % | 81 % | 76 % |

Die Beispiele 1 bis 4 zeigen zum Vergleich keine wesentlichen Unterschiede im Freisetzungsverhalten, die nahelegen wurden, daß in vivo über den Stand der Technik hinaus lang anhaltende Blutspiegel erzeugt werden.

Die erfindungsgemäßen Zubereitungen wurden auf ihre Bioverfügbarkeit an Versuchspersonen geprüft.

Beispiel 5

An jeweils vier gesunden freiwilligen Versuchspersonen im Alter zwischen 18 und 40 Jahren wurde die erfindungsgemäße Arzneiform nach Beispiel 1 getestet. Die Personen erhielten 2 Kapseln zu 20 mg als Einmalgabe = 40 mg Nifedipin bzw. 3 Kapseln zu 20 mg als Einmalgabe = 60 mg Nifedipin. In bestimmten Zeitabständen wurde Blut aus der Antekubitalvene entnommen und das Plasma mittels selektiver HPLC-Bestimmungsmethodeauf Nifedipin untersucht.

Ergebnisse:

| Zeit (h) | 0,5 | 1 | 2 | 3 | 4 | 6 | 9 | 12 | 24 | 36 |
|---|---|---|---|---|---|---|---|---|---|---|
| 40 mg Durchschn. aus N = 4 (ng/ml) | 4,5 | 6,9 | 8,3 | 6,3 | 6,5 | 4,7 | 5,8 | 6,6 | 4,6 | 1,8 |
| 60 mg Durchschn. aus N = 4 (ng/ml) | 8,9 | 10,4 | 12,6 | 9,9 | 8,6 | 11,1 | 8,7 | 10,1 | 7,7 | 2,5 |

Die gemessenen Plasmakonzentrationen zeigen, daß sich bei Einmalgabe von 40 bzw. 60 mg Nifedipin entgegen den Erwartungen nach den Stand der Technik keine Spitzenkonzentrationen ausbilden, sondern vielmehr gleichmäßige Werte (Plateau) über 24 Stunden.

Beispiel 6 ·

Ein männlicher Hypertoniker, 48 Jahre, Gewicht 87 kg mit einem morgendlichen Bluthochdruck vor Therapiebeginn von ca. 190 mm HG-Säule systolisch und ca. 115 mm HG-Säule diastolisch wurde mit einem nifedipinhaltigen Präparat nach dem Stand der Technik (Adalat® 20 retard) entsprechend der Dosierungsempfehlung mit 2 x 20 mg pro Tag im Abstand von 12 Stunden therapiert.

Eine ausreichende Blutdrucksenkung fand nicht statt, so daß die Dosis auf 3 x 20 mg pro Tag erhöht wurde. Nach einer 10-tägigen Therapiezeit wurde 3 Tage lang das Medikament abgesetzt, um die hypertone Ausgangslage wieder herzustellen. Anschließend wurde die Therapie mit 1 x 60 mg der Arzneiform gemäß Beispiel 4 wieder aufgenommen. Die Dosis wurde jeweils morgens gegeben. Folgende Blutdruckwerte wurden gemessen:

| | 1x60 mg Nifedipin gemäß Bsp. 4 | 3x20 mg Nifedipin Adalat® 20 mg retard |
|---|---|---|
| Blutdruck systolisch | 145-155 mm Hg | 150-165 mm Hg · |
| Blutdruck diastolisch | 90- 95 mm Hg | 90- 95 mm Hg |
| Pulsfrequenz . | Anstieg von 85 bis 90 - 93 im Tagesverlauf | Anstieg von 85 auf 100 - 105 jeweils 3 Std. nach Einnahme |
| Subjektive Nebenwirkungen | keine | Kopfdruck |

Der gemessene therapeutische Effekt der erfindungsgemäßen Form gegenüber einer Standardtherapie mit einem Standardarzneimittel bestätigt die Vorteilhaftigkeit der neuen Arzneizubereitung.

Weitere Beispiele sollen den erfindungsgemäßen Gegenstand erläutern:

Beispiel 7

| | |
|---|---|
| Nicardipinhydrochlorid | 18,0 g |
| Propylenglykol | 26,0 g |
| Polyethylenglykol (Mittl. Mol.-Gewicht 6000) | 132,0 g |
| Stearylalkohol | 36,0 g |
| Polyvinylpyrrolidon | 6,0 g |

Propylenglykol wird auf ca. 75° C erwärmt und darin das Nicardipin gelöst. Bei gleicher Temperatur werden nacheinander Polyethylenglykol und Stearylalkohol in die Lösung gegeben und geschmolzen. Der klaren Mischung wird Polyvinylpyrrolidon zugesetzt. Die Schmelze wird in Hartgelatinekapseln der Größe 0 zu 747 mg Füllgewicht, entsprechend 59 mg Nicardipin pro Kapsel, abgefüllt.

Beispiel 8

| | | |
|---|---|---|
| Nicardipinhydrochlorid | | 18,0 g |
| Propylenglykol | | 60,0 g |
| Polyethylenglykol (Mittl. Mol.-Gewicht 6000) | | 132,0 g |
| Stearylalkohol | | 72,0 g |

Propylenglykol wird auf ca. 75°C erwärmt und Nicardipin darin gelöst. In die Lösung werden bei gleicher Temperatur nacheinander Polyethylenglykol und Stearylalkohol eingerührt und geschmolzen. Die klare Schmelze wird in Hartgelatinekapseln der Größe 0 zu 727 mg Füllgewicht, entsprechend 47 mg Nicardipin pro Kapsel, abgefüllt.

In vitro-Freisetzungsraten

Das Freisetzungsverhalten des Wirkstoffes aus den Arzneiformen wurde bei konstantem pH-Wert von 1,5 geprüft. (Angaben in % der jeweils enthaltenen Wirkstoffmenge).

Tabelle Nr. 2

| | Beispiel 7 | Beispiel 8 |
|---|---|---|
| 1 Stunde | 47 % | 25 % |
| 2 Stunden | 68 % | 36 % |
| 3 Stunden | 80 % | 44 % |
| 4 Stunden | 87 % | 50 % |
| 5 Stunden | 92 % | 55 % |
| 6 Stunden | 95 % | 60 % |
| 7 Stunden | 97 % | 64 % |

Die Ergebnisse aus diesen beispielhaften Studien ermöglichen gegenüber dem Stand der Technik, Dihydropyridine, insbesondere die der genannten Art, in bislang unüblichen hohen Dosen einmal pro Tag (Einmaldosen) zu verabreichen.

Diese Einmaldosierungen lassen eine erhöhte Therapiesicherheit (Patienten Compliance) zu, vermindern die Nebenwirkungshäufigkeit und stellen nicht zuletzt durch die Einfachheit des Herstellverfahrens eine Bereicherung des Arzneimittelschatzes zur Behandlung koronarer Herzkrankheiten und des Bluthochdruckes dar.

Patentansprüche

1. Feste Arzneizubereitungen zur oralen Verabreichung, die Dihydropyridin(e) als Wirkstoff(e) in einer Dosis von 10 bis 240 mg in gelöstem, moleculardispersem Zustand in Polyethylenglykolen mit mittlerem Molekulargewicht von 6 000 bis 35 000 zusammen mit einem Fettalkohol oder Fettalkoholgemisch mit Kettenlängen von 6 bis 30 Kohlenstoffatomen enthalten, wobei das Verhältnis Dihydropyridin zu den Polyethylenglykolen 1 : 2 bis 1 : 50 und das Verhältnis Dihydropyridin zu Fettalkoholen 1 : 0,1 bis 1 : 10 beträgt.

2. Feste Arzneizubereitungen nach Anspruch 1, in der das Verhältnis Dihydropyridin zu den Polyethylenglykolen 1 : 4 bis 1 : 40 beträgt.

3. Feste Arzneizubereitungen nach Anspruch 1 oder 2, mit einer Dosis des Dihydropyridins von 30 bis 120 mg.

4. Feste Arzneizubereitungen nach Anspruch 1, 2 oder 3, mit einer Dosis des Dihydropyridins von 40 bis 80 mg.

5. Feste Arzneizubereitungen nach Anspruch 1 bis 4, dádurch gekennzeichnet, daß sie als Dihydropyridin Nifedipin enthalten.

6. Feste Arzneizubereitungen nach Anspruch 5, dadurch gekennzeichnet, daß Nifedipin in einer Dosis von 30 bis 120 enthalten ist.

7. Feste Arzneizubereitungen nach Anspruch 1 bis 4, dadurch gekennzeichnet, daß sie als Dihydropyridin Nimodipin, Nicardipin, Nitrendipin, Nisoldipin oder Felodipin enthalten.

8. Arzneizubereitungen nach einem der vorhergehenden Ansprüche, dadurch gekennzeichnet, daß sie Lösungsvermittler enthält, wobei das Verhältnis Dihydropyridin zu Lösungsvermittler 1 : 0,01 bis 1 : 3 beträgt.

9. Arzneizubereitungen nach Anspruch 1 bis 8 in Form von Granulaten, Tabletten, Pillen, überzogenen Tabletten, Kapseln, Sachets oder mehrschichtigen Tabletten.

10. Arzneizubereitungen nach Anspruch 1 bis 9 zur Behandlung von koronaren Herzkrankheiten und/oder des Bluthochdrucks.

**Claims**

1. Solid medicament preparations for oral administration, which contain dihydropyridine(s) as active compound(s) in a dose of 10 to 240 mg in dissolved, molecularly disperse state in polyethylene glycols of average molecular weight 6,000 to 35,000 together with a fatty alcohol or fatty alcohol mixture having chain lengths from 6 to 30 carbon atoms, where the ratio of dihydropyridine to the polyethylene glycols is 1:2 to 1:50 and the ratio of dihydropyridine to fatty alcohols is 1:0.1 to 1:10.

2. Solid medicament preparations according to Claim 1, in which the ratio of dihydropyridine to the polyethylene glycols is 1:4 to 1:40.

3. Solid medicament preparations according to Claim 1 or 2, containing a dose of the dihydropyridine of 30 to 120 mg.

4. Solid medicament preparations according to Claim 1, 2 or 3, containing a dose of the dihydropyridine of 40 to 80 mg.

5. Solid medicament preparations according to Claim 1 to 4, characterised in that they contain nifedipine as the dihydropyridine.

6. Solid medicament preparations according to Claim 5, characterised in that nifedipine is present in a dose of 30 to 120 mg.

7. Solid medicament preparations according to Claim 1 to 4, characterised in that they contain nimodipine, nicardipine, nitrendipine, nisoldipine or felodipine as the dihydropyridine.

8. Medicament preparations according to one of the preceding claims, characterised in that they contain solubilizers, the ratio of dihydropyridine to solubilizer being 1:0.01 to 1:3.

9. Medicament preparations according to Claim 1 to 8 in the form of granules, tablets, pills, coated tablets, capsules, sachets or multi-coated tablets.

10. Medicament preparations according to Claim 1 to 9 for the treatment of coronary heart diseases and/or of high blood pressure.

**Revendications**

1. Préparations pharmaceutiques solides pour administration orale, qui contiennent de la (des) dihydropyridine(s) comme substance(s) active(s) dans une dose de 10 à 240 mg à l'état dissous, en dispersion moléculaire, dans des polyéthylèneglycols de poids moléculaire moyen de 6.000 à 35.000 et un alcool gras ou un mélange d'alcool gras avec des longueurs de chaîne de 6 à 30 atomes C, le rapport dihydropyridine/polyéthylèneglycol étant de 1:2 à 1:50 et le rapport dihydropyridine/alcool gras de 1:0,1 à 1:10.

2. Préparations pharmaceutiques solides selon la revendication 1 caractérisées en ce que le rapport dihydropyridine/polyéthylèneglycols est de 1:4 à 1:40.

3. Préparations pharmaceutiques solides selon la revendication 1 ou 2, avec une dose de dihydropyridine de 30 à 120 mg.

4. Préparations pharmaceutiques solides selon les revendications 1, 2 ou 3 avec une dose de dihydropyridine de 40 à 80 mg.

5. Préparations pharmaceutiques solides selon une des revendications 1 à 4 caractérisées en ce qu'elles contiennent de la nifédipine comme dihydropyridine.

6. Préparations pharmaceutiques solides selon la revendication 5 caractérisées en ce que la nifédipine est contenue dans une dose de 30 à 120 mg.

7. Préparations pharmaceutiques solides selon une des revendications 1 à 4 caractérisées en ce qu'elles contiennent comme dihydropyridine de la nimodipine, de la nicardipine, de la nitrendipine, de la nisoldipine ou de la félodipine.

8. Préparations pharmaceutiques selon une des revendications précédentes caractérisées en ce qu'elles contiennent des agents solubilisants, le rapport dihydropyridine/agent solubilisant étant de 1:0,01 à 1:3.

9. Préparations pharmaceutiques selon une des revendications 1 à 8 sous forme de granulés, comprimés, pilules, comprimés dragéifiés, capsules, sachets ou comprimés multicouches.

10. Préparations pharmaceutiques selon une des revendications 1 à 9 pour le traitement des maladies coronariennes et/ou de l'hypertension artérielle.